# EUROPEAN PATENT APPLICATION

(11) **EP 4 576 108 A1**
(43) Date of publication of application: **25.06.2025**
(21) Application number: 24222352.7
(22) Date of filing: 20.12.2024
(51) Int. Cl.: G16H 10/60, G16H 40/63, G16H 40/67

(54) **MULTI-DEVICE PATIENT ENCOUNTERS**

(30) Priority: 21.12.2023 US 202363613716 P
(71) Applicant: Physio-Control, Inc., Redmond, WA 98052 (US)
(72) Inventor: FROLOV, Alexander, Redmond, WA 98052 (US); SKELTON, Dennis M., Redmond, WA 98052 (US); DUKE, Steven Barry, Redmond, WA 98052 (US); RUTZER, Mark, Redmond, WA 98052 (US); KILLEBREW, Mark G., Redmond, WA 98052 (US); PADMANABHA, Seshadri K., Redmond, WA 98052 (US); STEWART, David B., Redmond, WA 98052 (US); WOOTTEN, James, Redmond, WA 98052 (US); BALES, Robert, Redmond, WA 98052 (US); MACKIE, Richard, Redmond, WA 98052 (US); BEUNING, Gail R., Redmond, WA 98052 (US)
(74) Representative: V.O.

(57) **Abstract**

A method includes receiving, by a server, first patient data from a first device. The first patient data was collected by the first device during a patient encounter. The method includes receiving, from the first device, record-identifying information of a second device that was associated with the patient encounter. During the patient encounter, the record-identifying information was shared with the first device. The method include generating a request for second patient data collected by the second device during the patient encounter. The method includes sending the request for the second patient data to the second device based on the record-identifying information. The method includes receiving the second patient data from the second device after sending the request for the second patient data to the second device. The method includes generating a record for the patient encounter based on at least on the first patient data and the second patient data.

## Description

### Field

The present disclosure generally relates to collecting patient data, and more particularly, ensuring patient data collected from multiple devices is collected and sent to a server.

### Background

Different devices can be used during a patient encounter to collect patient data. For example, a first device can be used to collect first patient data, and a second device can be used to collect second patient data. In some scenarios, the first and second devices can send the first and second patient data, respectively, to a server. From there, the server can generate a record of the patient encounter and send the record to a treating facility, such as a hospital.
In some instances, after the patient encounter, there is a risk that all of the involved devices may not send corresponding patient data to the server. To illustrate, the first device can send the first patient data to the server when the first device has access to a centralized network. Upon receiving the first patient data, the server can generate a record for the patient encounter based on the first patient data. However, if the second device does not send the second patient data to the server, the record for the patient encounter may not reflect all of the patient data collected from the different devices during the patient encounter. In other words, the record for the patient encounter generated by the server may be incomplete.

### Summary

In one aspect, the present application discloses a server. The server includes a processor and a memory storing computer-executable instructions that, when executed by the processor, cause the processor to receive first patient data from a first device. The first patient data was collected by the first device during a patient encounter. The computer-executable instructions also cause the processor to receive, from the first device, record-identifying information of a second device that was associated with the patient encounter. During the patient encounter, the record-identifying information of the second device was shared with the first device. The computer-executable instructions also cause the processor to generate a request for second patient data collected by the second device during the patient encounter. The computer-executable instructions also cause the processor to send the request for the second patient data to the second device based on the record-identifying information of the second device. The computer-executable instructions also cause the processor to receive the second patient data from the second device after sending the request for the second patient data to the second device. The computer-executable instructions also cause the processor to generate a record for the patient encounter based at least on the first patient data and the second patient data.

In another aspect, the present application discloses a method. The method includes receiving, by a server, first patient data from a first device. The first patient data was collected by the first device during a patient encounter. The method also includes receiving, by the server and from the first device, record-identifying information of a second device that was associated with the patient encounter. During the patient encounter, the record-identifying information of the second device was shared with the first device. The method also include generating, by the server, a request for second patient data collected by the second device during the patient encounter. The method also includes sending, by the server, the request for the second patient data to the second device based on the record-identifying information of the second device. The method also includes receiving, by the server, the second patient data from the second device after sending the request for the second patient data to the second device. The method also includes generating, by the server, a record for the patient encounter based on at least on the first patient data and the second patient data.

In another aspect, the present application discloses a non-transitory computer-readable medium. The non-transitory computer-readable medium includes instructions that, when executed by a processor of a server, causes the processor to perform operations. The operations receiving first patient data from a first device. The first patient data was collected by the first device during a patient encounter. The operations also includes receiving, from the first device, record-identifying information of a second device that was associated with the patient encounter. During the patient encounter, the record-identifying information of the second device was shared with the first device. The operations also include generating a request for second patient data collected by the second device during the patient encounter. The operations also includes sending the request for the second patient data to the second device based on the record-identifying information of the second device. The operations also includes receiving the second patient data from the second device after sending the request for the second patient data to the second device. The operations also includes generating a record for the patient encounter based on at least on the first patient data and the second patient data.

The foregoing summary is illustrative only and is not intended to be in any way limiting. In addition to the illustrative aspects, embodiments, and features described above, further aspects, embodiments, and features will become apparent by reference to the figures and the following detailed description.

### Brief Description of the Drawings

A more complete understanding of embodiments of the present application may be derived by referring to the detailed description and claims when considered in conjunction with the following figures, wherein like reference numbers may refer to similar elements throughout the figures. The figures are provided to facilitate understanding of the disclosure without limiting the breadth, scope, scale, or applicability of the disclosure. The drawings are not necessarily made to scale.
Figure 1 illustrates a first instance of a patient encounter where multiple devices collect patient data, according to an exemplary embodiment;
Figure 2 illustrates a second instance of the patient encounter where multiple devices collect patient data, according to an exemplary embodiment;
Figure 3 illustrates an example of a device associated with the patient encounter providing information to a server, according to an exemplary embodiment;
Figure 4 illustrates an example of the server requesting patient data from a device associated with the patient encounter, according to an exemplary embodiment;
Figure 5 illustrates an example of the server requesting patient data from another device associated with the patient encounter, according to an exemplary embodiment;
Figure 6 illustrates a process for generating a patient encounter record, according to an exemplary embodiment; and
Figure 7 is a flowchart of a method for generating a patient encounter record based on patient data from multiple devices, according to an exemplary embodiment.

### Detailed Description

The following description describes techniques for ensuring patient data collected from multiple devices is collected and sent to a server. Different devices, such as medical devices and mobile devices, can be used during a patient encounter to provide medical treatment to a patient and/or collect patient data. As a non-limiting example, a defibrillator can be used to perform defibrillation operations on the patient, a patient monitoring device can be used to monitor vital signs of the patient, etc. Each device used during the patient encounter can collect different patient data. To illustrate, a first device (e.g., the defibrillator) can collect first patient data, such as cardiovascular information associated with the patient's heartbeat. Additionally, a second device (e.g., the patient monitoring device) can be used collect second patient data, such as respiratory information associated with the patient's breathing patterns.

In some scenarios, during the patient encounter, the first device and the second device may not be able to send (e.g., upload) the first and second patient data, respectively, to a server. As a non-limiting example, the patient encounter may occur at a location that does not have access to a centralized network, such as an Institute of Electrical and Electronics Engineers 802.11 ("WiFi") network or a cellular network. As a result, the first device and the second device may not be able to send data to the server during the patient encounter.

After the patient encounter, there is a risk that all of the involved devices may not send corresponding patient data to the server. To illustrate, the first device can send the first patient data to the server when the first device has access to a centralized network. Upon receiving the first patient data, the server can generate a record for the patient encounter based on the first patient data. However, if the second device does not send the second patient data to the server, the record for the patient encounter may not reflect all of the patient data collected from the different devices during the patient encounter. In other words, the record for the patient encounter generated by the server may be incomplete.

To ensure that the server can generate a complete record of the patient encounter, the devices that send patient data to the server can also send, to the server, record-identifying information of one or more other devices that participated in the patient encounter. To illustrate, during the patient encounter, the first device and the second device can share record-identifying information using a decentralized network, such as WiFi Direct, Bluetooth, etc. The record-identifying information can indicate a device serial number, a time of the patient encounter, a location of the patient encounter, identification information of the patient (e.g., a name of the patient, a date of birth of the patient, a physical appearance description of the patient, a description of the patient's injuries, etc.), or any other information usable to identify the patient encounter from other patient encounters. When the first device sends the first patient data to the server, the first device can also send the record-identifying information of the second device to the server.

In response to receiving the record-identifying information of the second device, the server can (i) generate a request for the second patient data collected by the second device during the patient encounter and (ii) send the request to the second device using the record-identifying information of the second device. Upon receiving the request from the server, the second device can send the second patient data to the server, and the server can update the record for the patient encounter based on the second patient data.

It should be understood that record-identifying information for additional devices that participated in the patient encounter can be sent to the server via the first or second device. As a non-limiting example, if a third device and the second device were at the patient encounter at a particular time and the first device was not at the patient encounter at the particular time, there stands a possibility that the first and third devices were never at the patient encounter together. As a result, the first device may not have record-identifying information of the third device. However, the second and third devices can share record-identifying information with one another using a decentralized network when the second and third devices are at the patient encounter together. In this scenario, when the second device sends the second patient data to the server (in response to the request from the server), the second device can also send the record-identifying information of the third device to the server. The server can use the record-identifying information of the third device to request, from the third device, third patient data collected by the third device during the patient encounter. Upon receiving the third patient data, the server can update the record for the patient encounter based on the third patient data.

Thus, the techniques described herein provide protocols that increase the likelihood that the server can collect patient data from all devices that collected patient data during the patient encounter.

The figures and the following description illustrate specific exemplary embodiments. It will be appreciated that those skilled in the art will be able to devise various arrangements that, although not explicitly described or shown herein, embody the principles described herein and are included within the scope of the claims that follow this description. Furthermore, any examples described herein are intended to aid in understanding the principles of the disclosure and are to be construed as being without limitation. As a result, this disclosure is not limited to the specific embodiments or examples described below, but by the claims and their equivalents.

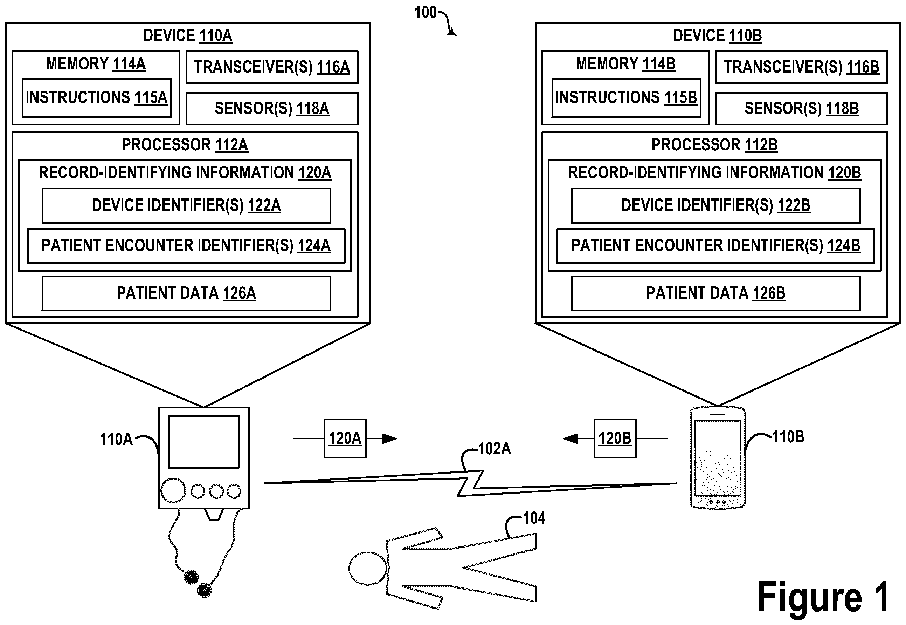

Particular implementations are described herein with reference to the drawings. In the description, common features may be designated by common reference numbers throughout the drawings. In some drawings, multiple instances of a particular type of feature are used. Although these features are physically and/or logically distinct, the same reference number is used for each, and the different instances are distinguished by addition of a letter to the reference number. When the features as a group or a type are referred to herein (e.g., when no particular one of the features is being referenced), the reference number is used without a distinguishing letter. However, when one particular feature of multiple features of the same type is referred to herein, the reference number is used with the distinguishing letter. For example, referring to Figure 1, devices are illustrated and associated with reference number 110. When referring to a particular one of the devices, such as the device 110A, the distinguishing letter "A" is used. However, when referring to any arbitrary one of the devices or to the devices as a group, the reference number 110 may be used without a distinguishing letter.

As used herein, various terminology is used for the purpose of describing particular implementations only and is not intended to be limiting. For example, the singular forms "a," "an," and "the" are intended to include the plural forms as well, unless the context clearly indicates otherwise. Further, the terms "comprise," "comprises," and "comprising" are used interchangeably with "include," "includes," or "including." Additionally, the term "wherein" is used interchangeably with the term "where." As used herein, "exemplary" indicates an example, an implementation, and/or an aspect, and should not be construed as limiting or as indicating a preference or a preferred implementation. As used herein, an ordinal term (e.g., "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). As used herein, the term "set" refers to a grouping of one or more elements, and the term "plurality" refers to multiple elements.

Referring to Figure 1, a first instance of a patient encounter 100 where multiple devices collect patient data is illustrated, in accordance with an exemplary embodiment. In particular, in Figure 1, a device 110A and a device 110B are used in the patient encounter 100 to collect patient data 126A, 126B, respectively, from a patient 104. In some implementations, the patient encounter 100 can correspond to an accident scene and the devices 110A, 110B can be used by paramedics to collect the patient data 126A, 126B.

In Figure 1, the device 110A is depicted as a medical device. In particular, in Figure 1, the device 110A is depicted as a defibrillator. However, it should be understood that a defibrillator is merely one non-limiting example of the device 110A. In other implementations, the device 110A can include any mobile (or stationary) device or smart sensor that can collect patient data 126A. The device 110A includes a processor 112A, a memory 114A coupled to the processor 112A, one or more transceivers 116A coupled to the processor 112A, and one or more sensors 118A coupled to the processor 112A. The memory 114A can be a non-transitory computer-readable medium that includes instructions 115A that are executable by the processor 112A to perform operations associated with the device 110A. The one or more transceivers 116A can include a Bluetooth transceiver, a WiFi transceiver, a cellular transceiver, or any other transceiver that connects to a corresponding network.

The sensors 118A can be configured to sense patient data 126A from the patient 104, and the processor 112A can be configured to collect the patient data 126A. The patient data 126A can include any medical data that can be obtained using a medical device, such as the device 110A. Non-limiting examples of the patient data 126A include heartrate activity of the patient 104, heart patterns of the patient 104, etc. In scenarios where the device 110A is not a defibrillator, the patient data 126A can include different data. As non-limiting examples, the patient data 126A can include oxygen levels of the patient 104, video of the patient 104 or the patient encounter 100, recorded audio, writings (e.g., charts, notes, etc.) generated by paramedics, etc.

At the time of the patient encounter 100, the processor 112A can be configured to generate record-identifying information 120A indicative of the patient encounter 100. The record-identifying information 120A includes one or more device identifiers 122A and one or more patient encounter identifiers 124A. The one or more device identifiers 122A can include a serial number of the device 110A or any other identifying information of the device 110A. The one or more patient encounter identifiers 124A can include information about the patient encounter 100. As non-limiting examples, the one or more patient encounter identifiers 124A can include a time of the patient encounter 100, a location of the patient encounter 100, identification information of the patient 104 (e.g., a name of the patient 104, a date of birth of the patient 104, a physical appearance description of the patient 104, a description of the patient's 104 injuries, etc.), or any other information usable to identify the patient encounter 100 from other patient encounters.

In Figure 1, the device 110B is depicted as a mobile device. As non-limiting examples, the device 110B can be a mobile phone, a laptop, personal digital assistant (PDA), tablets, etc. However, it should be understood that a mobile device is merely one non-limiting example of the device 110B. In other implementations, the device 110B can include any mobile (or stationary) device or smart sensor that can collect patient data 126B. The device 110B includes a processor 112B, a memory 114B coupled to the processor 112B, one or more transceivers 116B coupled to the processor 112B, and one or more sensors 118B coupled to the processor 112B. The memory 114B can be a non-transitory computer-readable medium that includes instructions 115B that are executable by the processor 112B to perform operations associated with the device 110B. The one or more transceivers 116B can include a Bluetooth transceiver, a WiFi transceiver, a cellular transceiver, or any other transceiver that connects to a corresponding network.

The sensors 118B can be configured to sense patient data 126B from the patient 104, and the processor 112B can be configured to collect the patient data 126B. The patient data 126B can include any medical data that can be obtained using sensors 118B on a mobile device 110B. Non-limiting examples of the patient data 126B include oxygen levels of the patient 104, heartrate activity of the patient 104, etc. The patient data 126B can also include video of the patient encounter 100 recorded by the device 110B, audio of the patient encounter 100 recorded by the device 110B, notes about the patient encounter 100 recorded by a paramedic, etc.

At the time of the patient encounter 100, the processor 112B can be configured to generate record-identifying information 120B indicative of the patient encounter 100. The record-identifying information 120B includes one or more device identifiers 122B and one or more patient encounter identifiers 124B. The one or more device identifiers 122B can include a serial number of the device 110B or any other identifying information of the device 110B. The one or more patient encounter identifiers 124B can include information about the patient encounter 100. As non-limiting examples, the one or more patient encounter identifiers 124B can include a time of the patient encounter 100, a location of the patient encounter 100, identification information of the patient 104 (e.g., a name of the patient 104, a date of birth of the patient 104, a physical appearance description of the patient 104, a description of the patient's 104 injuries, etc.), or any other information usable to identify the patient encounter 100 from other patient encounters.

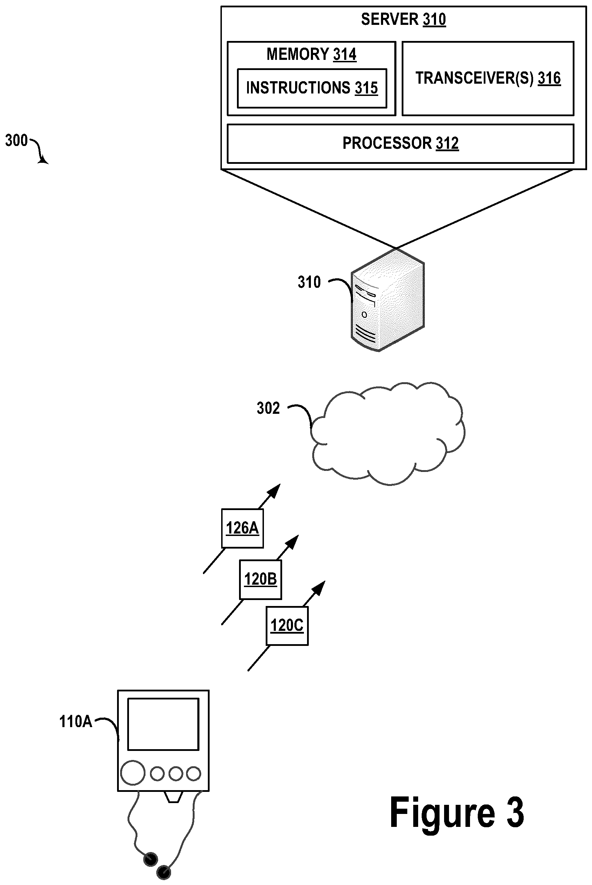

The devices 110A, 110B can exchange record-identifying information 120. For example, the device 110A can provide the device 110B with the record-identifying information 120A of the device 110A, and the device 110B can provide the device 110A with the record-identifying information 120B of the device 110B. To illustrate, a decentralized network 102A established between the devices 110A, 110B can be used to exchange record-identifying information 120A, 120B. In some implementations, the decentralized network 102A can correspond to a WiFi Direct network, a Bluetooth network, etc. As described in greater detail with respect to Figures 3-5, by exchanging record-identifying information 120, the devices 110 can assist a server 310 with generating a complete record of the patient encounter 100. In particular, the server 310 can use the record-identifying information 120 to (i) identify devices 110 that have not sent (e.g., uploaded) patient data 126 associated with the patient encounter 100 and (ii) request the patient data 126 from the devices 110.

In some scenarios, the devices 110A, 110B can use the decentralized network 102A to share the patient data 126A, 126B, respectively, with one another. In these scenarios, a single device 110 can upload, to the server 310, patient data 126 collected from multiple devices. To illustrate, the device 110A can share the patient data 126A collected during the patient encounter 100 with the device 110B. After receiving the patient data 126A from the device 110A, the device 110B can upload the patient data 126A and the patient data 126B to the server 310. Thus, in some scenarios, the devices 110A, 110B can merge the patient data 126 and send a single stream to the server 310 that includes all of the patient data 126 collected during the patient encounter.

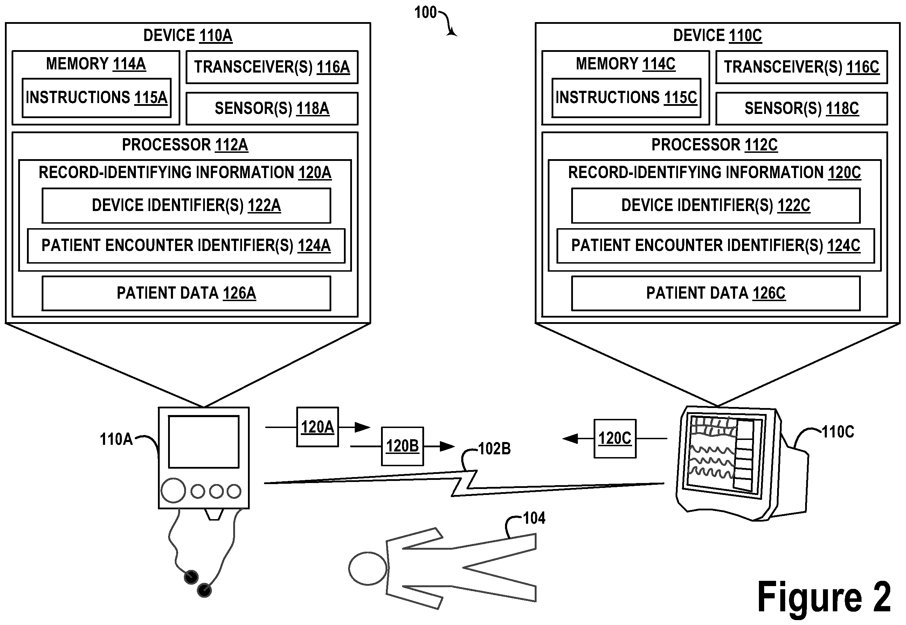

Referring to Figure 2, a second instance of the patient encounter 100 where multiple devices collect patient data is illustrated, in accordance with an exemplary embodiment. In particular, in Figure 2, the device 110A and a device 110C are used in the patient encounter 100 to collect patient data 126A, 126B, respectively, from the patient 104. Thus, in the second instance described with respect to Figure 2, the device 110B is no longer at the patient encounter 100 and the device 110C has arrived at the patient encounter 100.

In Figure 2, the device 110C is depicted as a patient monitor. However, it should be understood that a patient monitor is merely one non-limiting example of the device 110C. In other implementations, the device 110C can include any mobile (or stationary) device that can collect patient data 126C. In some scenarios, the device 110C can includes a smart sensor. The device 110C includes a processor 112C, a memory 114C coupled to the processor 112C, one or more transceivers 116C coupled to the processor 112C, and one or more sensors 118C coupled to the processor 112C. The memory 114C can be a non-transitory computer-readable medium that includes instructions 115C that are executable by the processor 112C to perform operations associated with the device 110C. The one or more transceivers 116C can include a Bluetooth transceiver, a WiFi transceiver, a cellular transceiver, or any other transceiver that connects to a corresponding network.

The sensors 118C can be configured to sense patient data 126C from the patient 104, and the processor 112C can be configured to collect the patient data 126C. The patient data 126C can include any medical data that can be obtained using sensors 118C on the device 110C. Non-limiting examples of the patient data 126C include oxygen levels of the patient 104, heartrate activity of the patient 104, etc.

At the time of the patient encounter 100, the processor 112C can be configured to generate record-identifying information 120C indicative of the patient encounter 100. The record-identifying information 120C includes one or more device identifiers 122C and one or more patient encounter identifiers 124C. The one or more device identifiers 122C can include a serial number of the device 110C or any other identifying information of the device 110C. The one or more patient encounter identifiers 124C can include information about the patient encounter 100. As non-limiting examples, the one or more patient encounter identifiers 124C can include a time of the patient encounter 100, a location of the patient encounter 100, identification information of the patient 104 (e.g., a name of the patient 104, a date of birth of the patient 104, a physical appearance description of the patient 104, a description of the patient's 104 injuries, etc.), or any other information usable to identify the patient encounter 100 from other patient encounters.

The devices 110A, 110C can exchange record-identifying information 120. For example, the device 110A can provide the device 110C with the record-identifying information 120A of the device 110A and the record-identifying information 120B of the device 110B, and the device 110C can provide the device 110A with the record-identifying information 120C of the device 110C. To illustrate, a decentralized network 102B established between the devices 110A, 110C can be used to exchange record-identifying information 120. In some implementations, the decentralized network 102B can correspond to a WiFi Direct network, a Bluetooth network, etc.

Thus, according to the techniques described with respect to Figure 2, the device 110C can receive the record-identifying information 120B of the device 110B if the device 110C and the device 110B were not at the patient encounter 100 at the same time. As described with respect to Figures 3-5, by exchanging record-identifying information 120, the devices 110 can assist the server 310 with generating a complete record of the patient encounter 100. In particular, the server 310 can use the record-identifying information 120 to (i) identify devices 110 that have not sent (e.g., uploaded) patient data 126 associated with the patient encounter 100 and (ii) request the patient data 126 from the devices 110.

In some embodiments, the decentralized network 102B used to share the record-identifying information 120 between the devices 110A, 110C can have a similar wireless protocol as the decentralized network 102A used to share the record-identifying information 120 between the devices 110A, 110B. As a non-limiting example, WiFi Direct can be used to share record-identifying information 120 in both instances. However, in other embodiments, the decentralized network 102B used to share the record-identifying information 120 between the devices 110A, 110C can have a different wireless protocol than the decentralized network 102A used to share the record-identifying information 120 between the devices 110A, 110B. As a non-limiting example, WiFi Direct can be used to share the record-identifying information 120 between the devices 110A, 110C, and Bluetooth can be used to share the record-identifying information 120 between the devices 110A, 110B.

Referring to Figure 3, an example 300 of a device associated with the patient encounter providing information to a server is illustrated, in accordance with an exemplary embodiment. For example, in Figure 3, the device 110A associated with the patient encounter 100 provides information to the server 310 to assist the server 310 with generating a record of the patient encounter 100.

The server 310 includes a processor 312, a memory 314 coupled to the processor 312, and one or more transceivers 316 coupled to the processor 312. The memory 314 can be a non-transitory computer-readable medium that includes instructions 315 that are executable by the processor 312 to perform operations associated with the server 310. The one or more transceivers 316 can a Bluetooth transceiver, a WiFi transceiver, a cellular transceiver, or any other transceiver that connects to a corresponding network, such as the network 302.

The device 110A can communicate with the server 310 using the network 302 (e.g., a centralized network). For example, the device 110A can send data to the cloud (e.g., the server 310 using the network 302), and the server 310 can send data to the device 110A using the network 302. According to one implementation, the network 302 is a WiFi network. According to one implementation, the network 302 is a cellular network, such as a public land mobile network (PLMN). The device 110A can use the network 302 to send the patient data 126A, the record-identifying information 120B of the device 110B, and the record-identifying information 120C of the device 110C to the server 310.

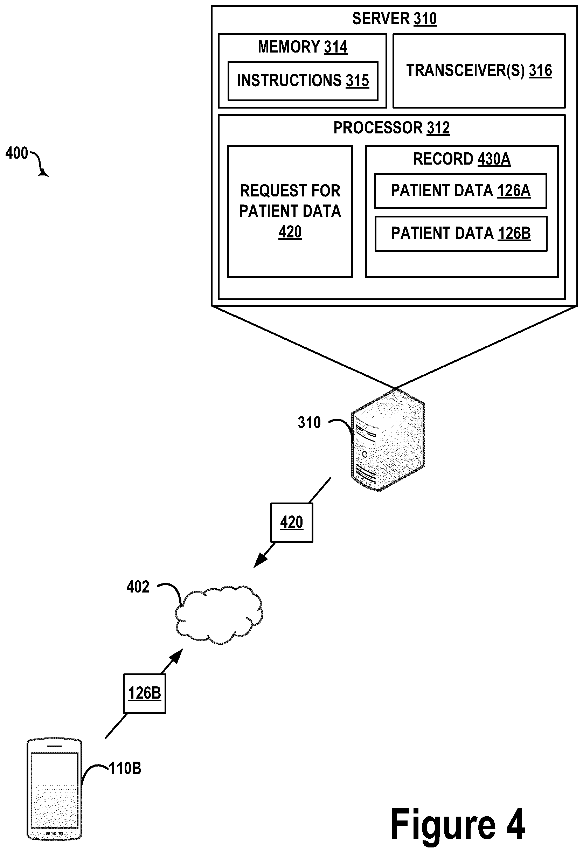

The server 310 can be configured to receive the patient data 126A from the device 110A via the network 302. The patient data 126A received by the server 310 corresponds to the patient data 126A collected by the device 110A during the patient encounter 100. The server 310 can also be configured to receive (i) the record-identifying information 120B of the device 110B from the device 110A via the network 302 and (ii) the record-identifying information 120C of the device 110C from the device 110A via the network 302. As described in greater detail with respect to Figures 4-5, by sharing the record-identifying information 120B, 120C of the other devices 110B, 110C associated with the patient encounter 100, if the other devices 110B, 110C did not send (e.g., upload) the patient data 126B, 126C collected during the patient encounter 100 to the server 310, the server 310 can request the other patient data 126B, 126C collected by the other devices 110B, 110C to generate a complete record of the patient encounter 100.

Referring to Figure 4, an example 400 of the server requesting patient data from a device associated with the patient encounter is illustrated, in accordance with an exemplary embodiment. For example, in Figure 4, the server 310 can request the patient data 126B from the device 110B.

The device 110B can communicate with the server 310 using a network 402 (e.g., a centralized network). For example, the device 110B can send data to the server 310 using the network 402, and the server 310 can send data to the device 110B using the network 402. According to one implementation, the network 402 is a WiFi network. According to one implementation, the network 402 is a cellular network, such as a PLMN. In some implementations, the network 402 is the same network as the network 302 of Figure 3. In other implementations, the network 402 is a different network than the network 302 of Figure 3.

In response to receiving the record-identifying information 120B of the device 110B from the device 110A (as illustrated in Figure 3), the server 310 can determine whether the device 110B has sent patient data 126B associated with the patient encounter 100 to the server 310. In response to a determination that the device 110B has not sent the patient data 126B associated with the patient encounter 100 to the server 310, the processor 312 of the server 310 can be configured to generate a request 420 for the patient data 126B. In particular, the processor 312 can use the device identifiers 122B in the record-identifying information 120B to identify the device 110B in the request 420, and the processor 312 can use the patient encounter identifiers 124B in the record-identifying information 120B to identify the specific patient encounter 100 in the request 420. The server 310 can send the request 420 to the device 110B. For example, the server 310 can use the transceiver 316 to send the request 420 to the device 110B via the network 402.

In response to receiving the request 420, the device 110B can send the patient data 126B to the server 310 via the network 402. Thus, the server 310 can be configured to receive the patient data 126B from the device 110B after sending the request 420 for the patient data 126B to the device 110B.

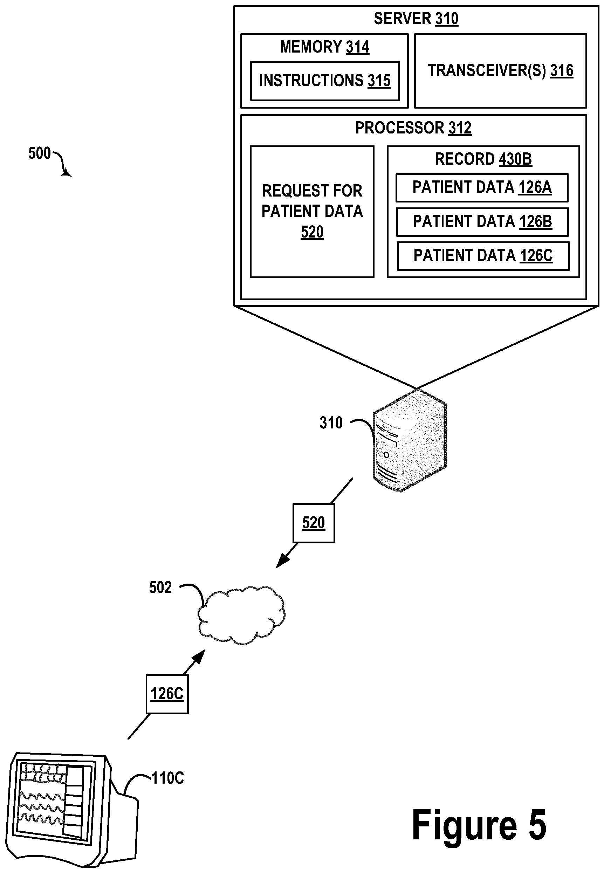

In response to receiving the patient data 126B from the device 110B, the server 310 can be configured to generate a record 430A for the patient encounter 100 based at least on the patient data 126A and the patient data 126B. As described in greater detail with respect to Figure 5, the server 310 can update the record 430A for the patient encounter 100 based on other patient data 126C collected by other devices 110C associated with the patient encounter 100.

Referring to Figure 5, an example 500 of the server requesting patient data from another device associated with the patient encounter is illustrated, in accordance with an exemplary embodiment. For example, in Figure 5, the server 310 can request the patient data 126C from the device 110C.

The device 110C can communicate with the server 310 using a network 502 (e.g., a centralized network). For example, the device 110C can send data to the server 310 using the network 502, and the server 310 can send data to the device 110C using the network 502. According to one implementation, the network 502 is a WiFi network. According to one implementation, the network 502 is a cellular network, such as a PLMN. In some implementations, the network 502 is the same network as one of the networks 302, 402. In other implementations, the network 502 is a different network than the networks 302, 402.

In response to receiving the record-identifying information 120C of the device 110C from the device 110A (as illustrated in Figure 3), the server 310 can determine whether the device 110C has sent patient data 126C associated with the patient encounter 100 to the server 310. In response to a determination that the device 110C has not sent the patient data 126C associated with the patient encounter 100 to the server 310, the processor 312 of the server 310 can be configured to generate a request 520 for the patient data 126C. In particular, the processor 312 can use the device identifiers 122C in the record-identifying information 120C to identify the device 110C in the request 520, and the processor 312 can use the patient encounter identifiers 124C in the record-identifying information 120C to identify the specific patient encounter 100 in the request 520. The server 310 can send the request 520 to the device 110C. For example, the server 310 can use the transceiver 316 to send the request 520 to the device 110C via the network 502.

In response to receiving the request 520, the device 110C can send the patient data 126C to the server 310 via the network 502. Thus, the server 310 can be configured to receive the patient data 126C from the device 110C after sending the request 520 for the patient data 126C to the device 110C. In response to receiving the patient data 126C from the device 110C, the server 310 can be configured to generate a record 430B (e.g., update the record 430A) for the patient encounter 100 based at least on the patient data 126A and the patient data 126B.

Thus, the techniques described with respect to Figures 1-5 provide protocols that increase the likelihood that the server 310 can retrieve patient data 126 from each device 110 that collected patient data 126 during the patient encounter 100. For example, as described with respect to Figures 1-5, three devices 110A-110C collected patient data 126 during the patient encounter 100, but only the device 110A sent its collected patient data 126A to the server 310 without a request from the server 310. However, because the other devices 110B, 110C shared their respective record-identifying information 120B, 120C with the device 110A, the device 110A is able to forward the record-identifying information 120B, 120C to the server 310 to notify the server 310 of the other devices 110B, 110C. In response to using the record-identifying information 120B, 120C to identify the other devices 110B, 110C that participated in the patient encounter 100, the server 310 can request the collected data 126B, 126C from the other devices 110B, 110C to generate a complete record 430B of the patient encounter 100.

Although the scenario depicted in Figures 1-5 illustrates the device 110A providing the server 310 with its collected patient data 126A and the record-identifying information 120B, 120C of the other devices 110B, 110C, it should be understood that the server 310 can retrieve the patient data 126 from each device 110 under different scenarios. As a non-limiting example, if the device 110C provides the server 310 with its collected patient data 126C and the record-identifying information 120A, 120B of the other devices 110A, 110B, the server 310 can use the record-identifying information 120A, 120B to send requests to the other devices 110A, 110B for their respective patient data 126A, 126B collected from the patient encounter 100.

As another non-limiting example, if the device 110B provides the server 310 with its collected patient data 126B and the record-identifying information 120A of the device 110A, the server 310 can use the record-identifying information 120A to send a request to the device 110A for the patient data 126A it collected from the patient encounter 100. From there, the device 110A can also send the record-identifying information 120C of the device 110C to the server 310. The server 310 can use the record-identifying information 120C to send, to the device 110C, the request 520 for the patient data 126C collected by the device 110C at the patient encounter 100.

The above-described techniques can also be used for error detection and correction. As a non-limiting example, a first device and a second device can be connected during a patient encounter, and each device can collect patient data during the patient encounter. Later, when the first device is powered off, it could be discovered that the first device was attached to a different patient than the second device and the users broke the association on the second device. When the first device sends data to the server, the information will indicate that there was a second device involved. However, when the second device sends the data, the server will learn that the association was done by mistake and will ensure that the data from the two devices is not linked during processing.

Additionally, the above-described techniques can also be implemented with accessories that have wired connection with one of the devices 110. For example, an accessory (or sensor) connected to an accessory port of one of the devices 11 can collect patient data 126. Later, the accessory (or sensor) can be connected to a data download tool to send the collected patient data 126 to the server 310.

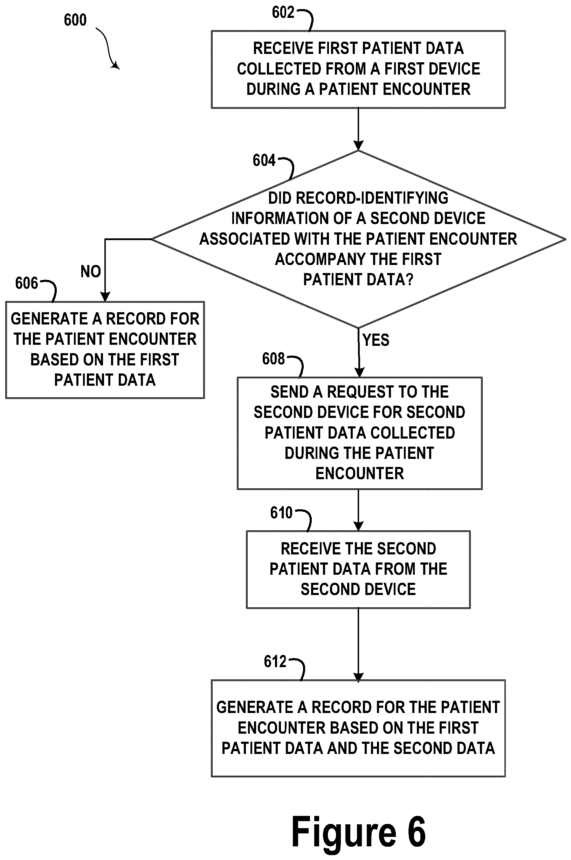

Figure 6 illustrates a process 600 for generating a patient encounter record, according to an exemplary embodiment. The process 600 can be performed by the server 310.

According to the process 600, at step 602, the server 310 can receive first patient data 126A collected from the first device 110A during the patient encounter 100. The first patient data 126A can be sent to the server 310 by the first device 110A via the network 302.

At step 604, the server 310 can determine whether record-identifying information 120B of a second device 110B associated with the patient encounter 100 accompanied the first patient data 126A. If record-identifying information 120B of a second device 110B did not accompany he first patient data 126A, at step 606, the server 310 can generate a record 430 for the patient encounter 100 based on the first patient data 126A. However, if record-identifying information 120B of a second device 110B did accompany the first patient data 126A, at step 608, the server 310 can send a request 420 to the second device 110B for second patient data 126B collected during the patient encounter 100 by the second device 110B.

At step 610, the server 310 can receive the second patient data 126B form the second device 110B. At step 612, the server 310 can generate a record 430 for the patient encounter 100 based on the first patient data 126A and the second patient data 126B.

The process 600 of Figure 6 provides protocols that increase the likelihood that the server 310 can retrieve patient data 126 from each device 110 that collected patient data 126 during the patient encounter 100. For example, as described with respect to Figures 1-5, three devices 110A-110C collected patient data 126 during the patient encounter 100, but only the device 110A sent its collected patient data 126A to the server 310 without a request from the server 310. However, because the other devices 110B, 110C shared their respective record-identifying information 120B, 120C with the device 110A, the device 110A is able to forward the record-identifying information 120B, 120C to the server 310 to notify the server 310 of the other devices 110B, 110C. In response to using the record-identifying information 120B, 120C to identify the other devices 110B, 110C that participated in the patient encounter 100, the server 310 can request the collected data 126B, 126C from the other devices 110B, 110C to generate a complete record 430B of the patient encounter 100.

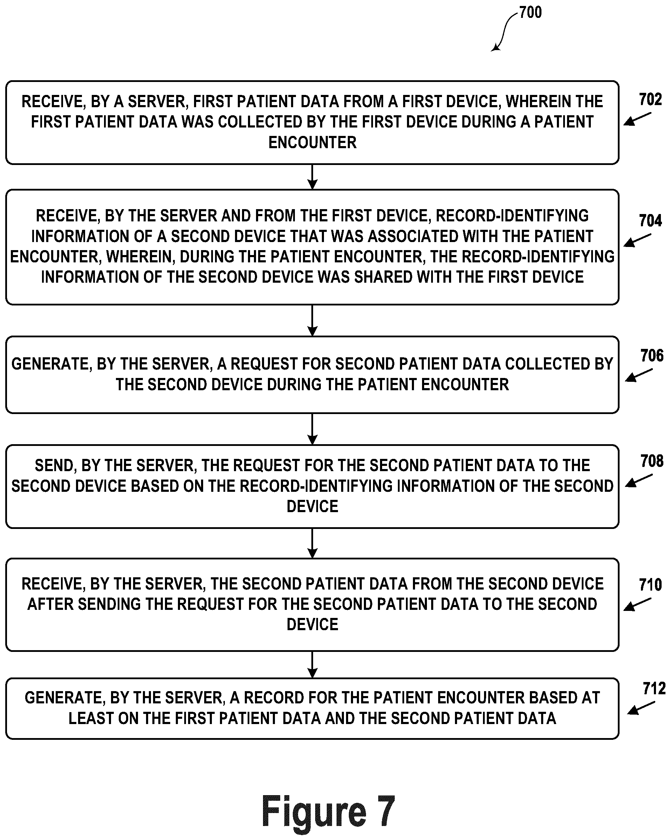

Figure 7 illustrates a flow chart of a method 700 generating a patient encounter record based on patient data from multiple devices, according to an exemplary embodiment. The method 700 can be performed by the server 310.

The method 700 includes receiving, by a server, first patient data from a first device, at block 702. The first patient data was collected by the first device during a patient encounter. For example, referring to Figures 1-5, the server 310 receives the first patient data 126A from the first device 110A. The first patient data 126A was collected by the first device 110A during the patient encounter 100.

The method 700 also includes receiving, by the server and from the first device, record-identifying information of a second device that was associated with the patient encounter, at block 704. During the patient encounter, the record-identifying information of the second device was shared with the first device. For example, referring to Figures 1-5, the server 310 receives, from the first device 110A, the record-identifying information 120B of the second device 110B that was associated with the patient encounter 100. During the patient encounter 100, the record-identifying information 120B of the second device 110B was shared with the first device 110A.

The method 700 also include generating, by the server, a request for second patient data collected by the second device during the patient encounter, at block 706. For example, referring to Figures 1-5, the server 310 generates the request 420 for the second patient data 126B collected by the second device 110B during the patient encounter 100.

The method 700 also includes sending, by the server, the request for the second patient data to the second device based on the record-identifying information of the second device, at block 708. For example, referring to Figures 1-5, the server 310 sends the request 420 for the second patient data 126B to the second device 110B based on the record-identifying information 120B of the second device 110B.

The method 700 also includes receiving, by the server, the second patient data from the second device after sending the request for the second patient data to the second device, at block 710. For example, referring to Figures 1-5, the server 310 receives the second patient data 126B from the second device 110B after sending the request 420 for the second patient data 126B to the second device 110B.

The method 700 also includes generating, by the server, a record for the patient encounter based at least on the first patient data and the second patient data, at block 712. For example, referring to Figures 1-5, the server 310 generates the record 430A for the patient encounter 100 based at least on the first patient data 126A and the second patient data 126B.

According to one implementation of the method 700, the record-identifying information 120B of the second device 110B was shared using a decentralized network 102A established between the first device 110A and the second device 110B.

According to one implementation, the method 700 includes receiving, by the server 310 and from the first device 110A or the second device 110B, record-identifying information of a third device 110C that was associated with the patient encounter 100. During the patient encounter 100, the record-identifying information 120C of the third device 110C was shared with at least one of the first device 110A or the second device 110B. The method 700 can also include generating, by the server 310, a request for the third patient data 126C collected by the third device 110C during the patient encounter 100. The method 700 can also include sending, by the server 310, the request for the third patient data 126C to the third device 110C based on the record-identifying information 120C of the third device 110C. The method 700 can also include receiving, by the server 310, the third patient data 126C from the third device 110C after sending the request for the third patient data 126C to the third device 110C. The record 430 for the patient encounter 100 is additionally generated based on the third patient data 126C.

According to one implementation of the method 700, the record-identifying information 120B of the second device 110B was shared using a first decentralized network 102A established between the first device 110A and the second device 110B, the record-identifying information 120C of the third device 110C was shared using a second decentralized network 102B established between the third device 110C and at least one of the first device 110A or the second device 110B. According to one implementation, the first decentralized network 102A and the second decentralized network 102B are based on a similar wireless protocol. According to one implementation, the first decentralized network 102A is based on a first wireless protocol, and the second decentralized network 102B is based on a second wireless protocol that is different from the first wireless protocol.

According to one implementation of the method 700, the first device 110A corresponds to a medical device or a mobile device that is operable to collect patient data. According to one implementation, the medical device includes a defibrillator or a patient monitor.

According to one implementation of the method 700, the request 420 for the second patient data 126B is sent to the second device 110B using a centralized network 402, and the second patient data 126B is received from the second device 110B using the centralized network 402.

The method 700 of Figure 7 provides protocols that increase the likelihood that the server 310 can retrieve patient data 126 from each device 110 that collected patient data 126 during the patient encounter 100. For example, as described with respect to Figures 1-5, three devices 110A-110C collected patient data 126 during the patient encounter 100, but only the device 110A sent its collected patient data 126A to the server 310 without a request from the server 310. However, because the other devices 110B, 110C shared their respective record-identifying information 120B, 120C with the device 110A, the device 110A is able to forward the record-identifying information 120B, 120C to the server 310 to notify the server 310 of the other devices 110B, 110C. In response to using the record-identifying information 120B, 120C to identify the other devices 110B, 110C that participated in the patient encounter 100, the server 310 can request the collected data 126B, 126C from the other devices 110B, 110C to generate a complete record 430B of the patient encounter 100.

Additionally, instances in this specification where one element is "coupled" to another element can include direct and indirect coupling. Direct coupling can be defined as one element coupled to and in some contact with another element. Indirect coupling can be defined as coupling between two elements not in direct contact with each other, but having one or more additional elements between the coupled elements. Further, as used herein, securing one element to another element can include direct securing and indirect securing. Additionally, as used herein, "adjacent" does not necessarily denote contact. For example, one element can be adjacent another element without being in contact with that element.

As used herein, a system, apparatus, structure, article, element, component, or hardware "configured to" perform a specified function is indeed capable of performing the specified function without any alteration, rather than merely having potential to perform the specified function after further modification. In other words, the system, apparatus, structure, article, element, component, or hardware "configured to" perform a specified function is specifically selected, created, implemented, utilized, programmed, and/or designed for the purpose of performing the specified function. As used herein, "configured to" denotes existing characteristics of a system, apparatus, structure, article, element, component, or hardware which enable the system, apparatus, structure, article, element, component, or hardware to perform the specified function without further modification. For purposes of this disclosure, a system, apparatus, structure, article, element, component, or hardware described as being "configured to" perform a particular function may additionally or alternatively be described as being "adapted to" and/or as being "operative to" perform that function.

The flow chart diagrams included herein are generally set forth as logical flow chart diagrams. As such, the depicted order and labeled steps are indicative of one embodiment of the presented method. Other steps and methods may be conceived that are equivalent in function, logic, or effect to one or more steps, or portions thereof, of the illustrated method. Additionally, the format and symbols employed are provided to explain the logical steps of the method and are understood not to limit the scope of the method. Although various arrow types and line types may be employed in the flow chart diagrams, they are understood not to limit the scope of the corresponding method. Indeed, some arrows or other connectors may be used to indicate only the logical flow of the method. For instance, an arrow may indicate a waiting or monitoring period of unspecified duration between enumerated steps of the depicted method. Additionally, the order in which a particular method occurs may or may not strictly adhere to the order of the corresponding steps shown.

Unless otherwise indicated, the terms "first," "second," etc. are used herein merely as labels, and are not intended to impose ordinal, positional, or hierarchical requirements on the items to which these terms refer. Moreover, reference to, e.g., a "second" item does not require or preclude the existence of, e.g., a "first" or lower-numbered item, and/or, e.g., a "third" or higher-numbered item.

While the systems and methods of operation have been described with reference to certain examples, it will be understood by those skilled in the art that various changes can be made and equivalents can be substituted without departing from the scope of the claims. Therefore, it is intended that the present methods and systems not be limited to the particular examples disclosed, but that the disclosed methods and systems include all embodiments falling within the scope of the appended claims.

## Claims

1. A server comprising:
a processor; and
a memory storing computer-executable instructions that, when executed by the processor, cause the processor to:
receive first patient data from a first device, wherein the first patient data was collected by the first device during a patient encounter;
receive, from the first device, record-identifying information of a second device that was associated with the patient encounter, wherein, during the patient encounter, the record-identifying information of the second device was shared with the first device;
generate a request for second patient data collected by the second device during the patient encounter;
send the request for the second patient data to the second device based on the record-identifying information of the second device;
receive the second patient data from the second device after sending the request for the second patient data to the second device; and
generate a record for the patient encounter based at least on the first patient data and the second patient data.

2. The server of claim 1, wherein the record-identifying information of the second device was shared using a decentralized network established between the first device and the second device.

3. The server of claim 1, wherein the computer-executable instructions further cause the processor to:
receive, from the first device or the second device, record-identifying information of a third device that was associated with the patient encounter, wherein, during the patient encounter, the record-identifying information of the third device was shared with at least one of the first device or the second device;
generate a request for third patient data collected by the third device during the patient encounter;
send the request for the third patient data to the third device based on the record-identifying information of the third device; and
receive the third patient data from the third device after sending the request for the third patient data to the third device,
wherein the record for the patient encounter is additionally generated based on the third patient data.

4. The server of claim 3, wherein the record-identifying information of the second device was shared using a first decentralized network established between the first device and the second device, and wherein the record-identifying information of the third device was shared using a second decentralized network established between the third device and at least one of the first device or the second device.

5. The server of claim 4, wherein the first decentralized network and the second decentralized network are based on a similar wireless protocol, or optionally wherein the first decentralized network is based on a first wireless protocol, and wherein the second decentralized network is based on a second wireless protocol that is different from the first wireless protocol.

6. The server of claim 1, wherein the first device corresponds to a medical device or a mobile device that is operable to collect patient data, wherein optionally the medical device includes a defibrillator or a patient monitor.

7. The server of claim 1, wherein the request for the second patient data is sent to the second device using a centralized network, and wherein the second patient data is received from the second device using the centralized network.

8. A method comprising:
receiving, by a server, first patient data from a first device, wherein the first patient data was collected by the first device during a patient encounter;
receiving, by the server and from the first device, record-identifying information of a second device that was associated with the patient encounter, wherein, during the patient encounter, the record-identifying information of the second device was shared with the first device;
generating, by the server, a request for second patient data collected by the second device during the patient encounter;
sending, by the server, the request for the second patient data to the second device based on the record-identifying information of the second device;
receiving, by the server, the second patient data from the second device after sending the request for the second patient data to the second device; and
generating, by the server, a record for the patient encounter based at least on the first patient data and the second patient data.

9. The method of claim 8, wherein the record-identifying information of the second device was shared using a decentralized network established between the first device and the second device.

10. The method of claim 8, further comprising:
receiving, by the server and from the first device or the second device, record-identifying information of a third device that was associated with the patient encounter, wherein, during the patient encounter, the record-identifying information of the third device was shared with at least one of the first device or the second device;
generating, by the server, a request for third patient data collected by the third device during the patient encounter;
sending, by the server, the request for the third patient data to the third device based on the record-identifying information of the third device; and
receiving, by the server, the third patient data from the third device after sending the request for the third patient data to the third device,
wherein the record for the patient encounter is additionally generated based on the third patient data.

11. The method of claim 10, wherein the record-identifying information of the second device was shared using a first decentralized network established between the first device and the second device, and wherein the record-identifying information of the third device was shared using a second decentralized network established between the third device and at least one of the first device or the second device.

12. The method of claim 11, wherein the first decentralized network and the second decentralized network are based on a similar wireless protocol, or optionally wherein the first decentralized network is based on a first wireless protocol, and wherein the second decentralized network is based on a second wireless protocol that is different from the first wireless protocol.

13. The method of claim 8, wherein the first device corresponds to a medical device or a mobile device that is operable to collect patient data, wherein optionally the medical device includes a defibrillator or a patient monitor.

14. The method of claim 8, wherein the request for the second patient data is sent to the second device using a centralized network, and wherein the second patient data is received from the second device using the centralized network.

15. A non-transitory computer-readable medium comprising instructions that, when executed by a processor of a server, causes the processor to perform operations comprising:
receiving first patient data from a first device, wherein the first patient data was collected by the first device during a patient encounter;
receiving, from the first device, record-identifying information of a second device that was associated with the patient encounter, wherein, during the patient encounter, the record-identifying information of the second device was shared with the first device;
generating a request for second patient data collected by the second device during the patient encounter;
sending the request for the second patient data to the second device based on the record-identifying information of the second device;
receiving the second patient data from the second device after sending the request for the second patient data to the second device; and
generating a record for the patient encounter based at least on the first patient data and the second patient data,
wherein optionally the first device corresponds to a medical device or a mobile device that is operable to collect patient data.
